# EUROPEAN PATENT SPECIFICATION

(11) **EP 1 465 876 B1**
(45) Date of publication and mention of the grant of the patent: **05.11.2008**
(21) Application number: 02805778.4
(22) Date of filing: 30.12.2002
(51) Int. Cl.: C07D 239/553, C07F 15/00, A61K 31/506, A61P 35/00, C07D 239/54

(54) **LONG CHAIN FATTY ALCOHOL SUBSTITUENTS IN ANTINEOPLASTIC AGENTS**
LANGKETTIGE FETTALKOHOLSUBSTITUENTEN IN MITTELN GEGEN KREBS
SUBSTITUANTS D'ALCOOL GRAS A CHAINE LONGUE DANS DES AGENTS ANTINEOPLASIQUES

(30) Priority: 31.12.2001 EP 01131049
(43) Date of publication of application: 13.10.2004
(73) Proprietor: Pfaendler, Hans Rudolf, 82131 Stockdorf (DE)
(72) Inventor: PFAENDLER, Hans, Rudolf, 82131 Stockdorf (DE); KLINGL, Alexander, 22525 Hamburg (DE)
(86) International application number: PCT/EP2002/014809
(87) International publication number: WO 2003/055864

(56) References cited:
- EP-A- 0 167 310
- WO-A-01/36431
- DE-A- 2 854 008
- DE-A- 3 432 320
- LISHENG CAI: "Synthesis and in vitro antitumor activity of oligonucleotide-tethered and related Platinum complexes" J. MED. CHEM., vol. 44, no. 18, 2001, pages 2959-2965, XP002196706 cited in the application
- KOKOTOS G ET AL: "Synthesis and in vitro cytotoxicity of lipophilic platinum(II) complexes" BIOORGANIC & MEDICINAL CHEMISTRY LETTERS, OXFORD, GB, vol. 8, no. 12, 16 June 1998 (1998-06-16), pages 1525-1530, XP004137077 ISSN: 0960-894X cited in the application

## Description

This invention relates to novel antineoplastic effective long chain hydroxyalkyl and hydroxyalkenyl compounds of the general formulae I, II and III 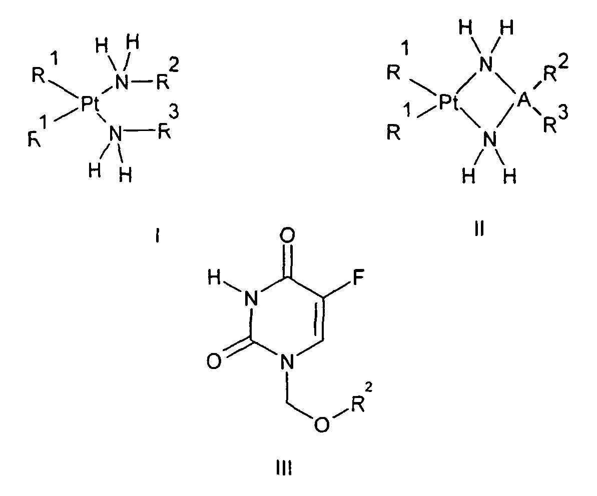 and their pharmaceutically acceptable salts, ester and prodrug derivatives wherein R¹ are either the same or different and are customary pharmaceutically acceptable inorganic or organic leaving groups, R² is an ω-hydroxyalkyl, (ω-hydroxy)alkenyl, (ω-(2,3-dihydroxypropyloxy)alkyl or an (ω-(2,3-dihydroxypropyloxy))alkenyl group with 5 to 30 carbon atoms and R³ is selected from hydrogen, C₁ - C₆- alkyl, C₃ - C₆- cycloalkyl, ω-hydroxyalkyl, (ω-hydroxy)alkenyl, (ω-(2,3-dihydroxypropyloxy)alkyl or an (ω-(2,3-dihydroxypropyloxy))alkenyl and where the foregoing alcoholic molecular parts are unbranched and have 5 to 30 carbon atoms and where A is 1,2-dimethylene, 1,3-trimethylene, 1,2-cyclopentylene or 1,2-cyclohexylene.

The substituents R² and R³, attached to the bridging unit A, can occur at any carbon atom of A.

Among the numerous antineoplastic agents used in the therapy of cancer, cisplatin and 5-fluorouracil are the most prominent ones. Since the discovery of their antitumor activity numerous of their analogues have been synthesized and evaluated. The target is to find chemotherapeutics with diminished side effects. The classes of antineoplastic agents is described in "Cancer Chemotherapeutic Agents", W. O. Foye, ed., Am. Chem. Soc. Professional Reference Book, Am. Chem. Soc., Washington, DC, 1995. Within the platinum(II) complexes some of these derivatives are less cytotoxic than cisplatin and are also used widely in cancer treatment. A description of such individual compounds and of the general aspects of platinum(II) cancerostatic agents is, for example, given in Merck Index, 13th ed., 1832, 2341, 6981 and in Angew. Chem. Int. Ed. Engl. 1987, 26, 615.
References to 5-fluorouracil and derivatives are given, for example, in Merck Index, 13th ed., 4208 and 1856.

Antineoplastic agents are not only cytotoxic agents against neoplastic cells but also to normal cells, resulting in severe side effects, very often precluding a successful treatment of cancer. This is not only given for the widely used platinum(II) complexes and for 5-fluorouracil but also to all other effective anticancer drugs.

It is an objective of the present patent application to provide novel antineoplastic agents which are more selective, i. e. which target neoplastic cells more specifically than antineoplastic agents of prior art do.

In order to target antineoplastic cells selectively, improved antineoplastic agent have to be designed, respecting the differences between tumor cells and their non-malignant counterparts.

Such an outstanding difference between cancerous tumour cells and non-malignant cells lies in the character of their lipids. The neoplastic tissues of animal and human contain increased levels of ether lipids, that cannot be metabolized, because neoplastic cells lack an enzyme called desaturase (*O-*alkylglycerol monooxygenase). In contrast, the non-malignant cells, containing the enzyme, can metabolize these uncommon lipids to glycerol and fatty acids via the plasmalogen (enol ether lipid) pathway. Therefore, non-malignant cells contain very low levels of ether lipids. This striking difference in ether lipid metabolism has also allowed to detect tumor cells by thin layer chromatography of the fatty alcohols, obtained from their lipids. Moreover, the content of non-esterified fatty alcohols was found to be approx. 30fold higher in neoplasms when compared to normal tissues. A comprehensive description about this subject is given in F. Snyder, "Ether Lipids", Academic Press, New York, London, pp. 273 - 295 (1972).

This concept has also been used to explain the relative cytotoxicity of alkyllysophospholipids (J. Koetting et al., Fat Sci. Technol. 90 (1988), 345 - 351).

Therefore, the compounds I, II and III according to the invention, contain one or two very specific long chain fatty alcohols, in order to allow the incorporation into ether lipids of malignant and non-malignant tissue.

Alternatively, they contain one or two very specific alkyl or alkenyl substituents having the terminal glyceryl ether moiety already incorporated by chemical synthesis. Apart from their better solubility in water, the obvious advantage of these latter compounds is that they do not require an additional step in the biosynthesis of the specific ether lipids. Consequently, within the glyceryl ether containing compounds according to the invention, long alkyl chains are not required. Within the platinum antineoplastic agents and the fluorouracil derivatives such glyceryl ether containing compounds have never been prepared nor investigated. Due to the above-mentioned difference in ether lipid metabolism it is to expect that, after incorporation of the compounds I, II and III, the resulting ether lipids are metabolized faster in non-malignant tissue relative to cancerous tumour tissue. They favour increased and long lasting levels of ether lipids predominantly in cancerous tumour tissue, thus allowing a selective treatment of neoplasms. Alternatively, in non-malignant tissues, after application of the compounds according to the invention, the incorporated glyceryl ether moiety can be metabolized to glycerol and carboxyalkylamine platinum (II) complexes, following the normal biological oxidation of ether lipids. Most important, such carboxy bearing amine-platinum (II) complexes are known to be of low toxicity (with glycine-platinum (II) complexes see Germ. Pat. Appl. DE 3128144). Consequently, a substantially reduced toxicity of the compounds according to the invention to non-malignant tissues is rationalized.

The antineoplastic activity of the compounds I, II and III according to the invention was determined in vitro against the cell lines MHEC 5T, LLC and EA.hy 926. Three representative compounds showed inhibition concentrations in the same order of magnitude as a clinically used antineoplastic agent.

Very recently, lipophilic anticancer agents have become known (Bioorg. Med. Chem. Lett. 1998, 1525 - 1530) within the platinum(II) complexes. Although the lipophilicity of these interesting compounds also favour the intracellular drug accumulation, a selective activity in tumor tissue cannot be rationalized, since these compounds of prior art lack a terminal ω-hydroxyalkyl group required for the incorporation into ether lipids.

3-Hydroxypropyl-1,2-diamine and bis-4-hydroxybutylamine platinum(II) complexes have become known (Chem. Pharm. Bull. 1983, 31, 1469 - 73 and J. Med. Chem. 2001, 44, 2959 - 2965). Again, the relatively short hydroxyalkyl chains disfavour a selective incorporation into the ether lipids of tumours.

A prerequisite for the successful application of a neoplastic agent is the incorporation into the tumour tissue. Referring to long chain alkyl and alkenyl alcohol groups as well as their glyceryl ethers it was found that they are indeed all incorporated efficiently into the liver of rats (FEBS Lett. 1971, 12, 217 - 220).

It is well known from the biosynthesis of triacylglycerols that natural fatty acids with even C-numbers are predominantly incorporated into these ester lipids. However, with fatty alcohols this is not given. It was found that the unnatural C₁₇ fatty alcohols, as glyceryl ethers, were incorporated well in all tissues of young rats (FEBS Lett.1988, 227, 187 - 190). Available evidence indicates that dietary 1 -*O*-heptadecyl-*sn*-glycerol is incorporated without the cleavage of the ether bond.

Therefore, not only the compounds I, II and III according to the invention that have even C-numbers in their alkyl or alkenyl molecular parts are useful as potential antineoplastic agents, but also the unnatural odd-numbered compounds. The c-chain lengths of the hydroxyl containing alkyl or alkenyl molecular parts are 5 to 30. The preferred range is 7 to 20 carbon atoms. Especially preferred are those with 12, 16, 17 or 18 carbon atoms. The preferred carbon numbers of the glyceryl containing substituents R² and R³ are 5 to 20 in total.

The C=C double bond within the alkenyl molecular parts of the compounds I, II and III according to the invention can be at any internal posistion. Preferred are compounds which have the double bond in the 9-position, related to oleic alcohol. Especially preferred are compounds wherein the alkenyl molecular part is 9-octadecenyl.

The geometry of the double bond can be cis or trans. The preferred geometry is cis.

It is an objective of the present invention to provide a novel class of medicaments for cancer therapy, that are more selective towards neoplastic cells than known antineoplastic agents. They differ from compounds known from prior art in that they contain very specific long chain alcohol residues or their glyceryl ethers forming a conjugate.

Therefore the present invention has the objective of providing a new and selective class of antineoplastic agents, which is important in veterinary and human therapy.

The new compounds I and II according to the invention are valuable antineoplastic agents. They can be used against many kinds of cancers, e. g. skin cancer, breast cancer, colon cancer and especially liver cancer.

The pharmaceutically acceptable customary leaving groups R¹ are known from prior art. These groups are known to be reactive leaving groups. They are variable within a wide range in the compounds I and II according to the invention, provided they are stable enough and soluble enough in water to allow the pharmaceutical application. It is known that these ligands exchange or are hydrolyzed in vivo. For example the chlorine atoms in cisplatin are hydrolyzed into hydroxyl groups in aqueous solutions. The groups R¹ can be derived from inorganic or organic acids. They can be separate groups or linked into a five- or six-membered ring. Examples for pharmaceutically acceptable inorganic groups R¹ are chloro, bromo, nitrato, thiocyanato, sulfato and for organic pharmaceutically acceptable groups groups are bis-nonadecanoato, 1,1-cyclopropane-dicarboxylato, 1,1-cyclobutane-dicarboxylato, hydroxyacetato or oxalato and the like. The requirements of the groups R¹ are known in the art and discussed, for example, in Angew. Chem. 1987, 99, 632 - 641 or Chem. i. u. Zeit, 1983, 6, 190 - 199.

The terminal substituents of the groups R² are hydroxyl or 2,3-dihydroxypropyloxy. The compounds containing the latter side chains are more soluble in water, due to the two hydroxyl groups per side chain. They already contain the glyceryl moiety allowing in-vivo formation of the diacylglyceryl ethers or their related phospholipids. The aforementioned hydroxy groups can also be esterified with lower alkyl carboxylic acids, preferably acetic acid. Such acylated derivatives of the compounds according to the invention can act as prodrugs, since they are deacylated easily by natural esterases in vivo.

Pharmaceutically acceptable salts are inorganic or organic salts which are known per se.

Pharmaceutically acceptable ester derivatives are either relatively stable groups such as fatty acid residues or hydrolyzable groups allowing the liberation of the parent alcohols or glyceryl ethers in vivo. Examples for such ester derivatives are acetates or formates and the like. Pharmaceutically acceptable ester derivatives also include those derived from dihydroxy platinum(IV) derivatives. These ester derivatives favour oral resorbability and act as prodrugs of the platinum (II) complexes according to the invention. Examples are formates acetates or butyrates. Such platinum prodrugs are known in the art and described e. g. in L.R. Kelland, "Cisplatin" (B. Lippert ed.), Verlag Helvetica Chimica Acta, Zurich and Wiley, Weilheim 1999, p. 497 - 521. But the dihydroxy platinum (IV) derivatives of the compounds according to the invention can also act as prodrugs themselves. They are metabolized in vivo to the antineoplastic Pt (II) complexes. Dihydroxy platinum (IV) complexes as antineoplastic agents are also described, e. g. in Ger. Offen. (1977) DE 2715492 19771020.

The principle of incorporating very specific long chain hydroxyalkyl, hydroxyalkenyl, 2,3-dihydroxypropyloxyalkyl or 2,3-dihydroxypropyloxyalkenyl groups in order to obtain tumour selective antineoplastic agents, as exemplified in the classes of platinum(II) or fluorouracil derivatives, obviously can also be applied to other customary antineoplastic agents leading to other tumour selective conjugates. Such agents which can be modified into the desired conjugates are, for example, listed in in "Cancer Chemotherapeutic Agents", W. O. Foye, ed., Am. Chem. Soc. Professional Reference Book, Am. Chem. Soc., Washington, DC, 1995, pp. 40 - 41 or in Merck Index, 13th ed. 4926 (2001). The above-mentioned principle is a novel solution of wide applicability to one of the most important problems in cancer therapy.

The compounds I, II and III according to the invention can exist in several stereochemical forms. With respect to the platinum ligands of compounds I or II the cis-geometry is preferred. With respect to the hydroxyalkyl and hydroxyalkenyl groups of compounds II both individual (R) and (S)-enantiomers are useful or the racemic mixture can be used. With respect to the glyceryl residue of compounds I, II and III also the (R) or (S)-configuration or a racemic mixture can be used to achieve the incorporation in tissue, although the individual stereoisomers are preferred.

The compounds I, II and III according to the invention may be used alone or together with other active components in any of a large number of pharmaceutical preparations. These preparations are known per se. They can be used in capsule form or in tablets, powders or liquid solutions or as suspensions or elixirs. They can be administered orally, intravenously or intramuscularly.

The preparations are preferably administered in a form which is suitable for absorption through the gastrointestinal tract. Tablets and capsules for oral administration may be in dose unit form and can contain customary medicament excipients, such as binders, for example syrup, gum arabic, sorbitol or polyvinylpyrrolidinone, fillers, for example lactose, sugar, maize starch, calcium phosphate, sorbitol or glycine, lubricants, for example magnesium stearate, talc, polyethylene glycol or silica, disintegrants for example potato starch, or acceptable wetting agents such as sodium lauryl sulfate. The tablets may be coated by processes which are known per se. Oral liquid preparations can be in the form of aqueous or oily suspensions, solutions, emulsions, syrups, elixirs and the like or can exist as dry product, for example for reconstitution before using water or other suitable excipients. Liquid preparations of this type can contain additives which are known per se, such as suspending agents, for example sorbitol syrup, methylcellulose, glucose/sugar syrup, gelatin, hydroxyethylcellulose, carboxymethylcellulose, aluminum strarate gel or hydrogenated edible oils, for example almond oil, fractionated coconut oil, oily esters, propylene glycol or ethyl alcohol, preservatives, for example methyl or propyl p-hydroxybenzoate or sorbic acid. Suppositories contain suppository bases which are known per se, for example cocoa butter or other glycerides.

The preparation for injection ca be in dose unit form in ampoules or in containers containing several doses along with an added preservative. The preparations can be in the form of suspensions, solutions or emulsions in oily or aqueous excipients, and they may contain formulation agents such as suspending agents, stabilizers and/or dispersants. Alternatively, the active component may be in powder form for reconstitution before using a suitalbe exipient, for example sterile, pyrogen-free water or physiological sodium chloride solution.

The preparations can also be in suitable form for absorption through the muscous membranes of the nose and of the throat tissue, and can be in the form of powders or liquid sprays or inhalants, sucking sweets, as throat paints, etc. Topical applications can exist or to be formulated in hydrophobic vehicles as ointments, creams, lotions, paints, powders etc.

The preparations according to the invention can contain, in addition to the excipient, other components such as stabilizers, binders, antioxidants, preservatives, lubricants, suspending agents, viscosity control agents or flavours or the like.

In addition, the preparations may contain one or more active neoplastic agents, e.g. methothrexate or the like.

The preparations according to the invention can be administered in various unit dose forms, for example in solid or liquid dose forms which can be taken orally. The preparations can contain 0.1 to 99 % of active material per unit dose, either in solid or in liquid form. The preferred range is about 10 to 60 %. The preparations generally contain 15 to about 1000 mg of active component but it is generally preferred to use a dose amount in the range about 50 to 500 mg. In the case of parenteral administration, the unit dose is normally the pure compound in a sterile water solution or in the form of a soluble powder, which may be dissolved.

The compounds I, II ans III according to the invention are prepared by conventional methods in few steps from available starting materials. The reaction schemes, leading to I, II and III are shown in the Example Section of the specification.

The following examples below illustrate the products, processes and preparations according to the invention.

### Example 1

### Preparation of cis-(Bis-(6-amine-1-hexanol))-dichloroplatinum(II)) (1a)

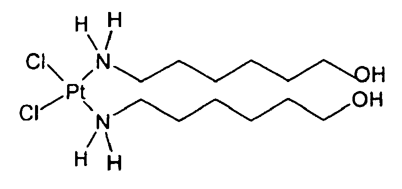

In a 25 ml flask fitted with a magnetic stirrer to 6-aminohexanol (148 mg, 1.26 mmol) 1 N aqueous hydrogen chloride (1.25 ml, 1.25 mmol) was added and the mixture stirred at a bath temperature of 65 °C where upon a clear solution resulted. Subsequently was added a solution of K₂PtCl₄ (200 mg, 0.48 mmol) in water (1.0 ml). Within 3 h, 1 N aqueous NaOH was added dropwise where upon the red solution turned pale yellow with the formation of a beige precipitate. In order to avoid the formation of black platinum hydroxide, special care was taken not to allow the reaction medium to become alkaline. After addition of 0.96 ml of 1 N NaOH (0.96 mmol), the suspension had pH 6 and it was allowed to stand at 0 °C for 10 min. The precipitate was collected by centrifugation. It was washed three times with cold 5 % sodium chloride solution (3 ml) and finally with cold water. The product was dried in high vacuum affording a beige powder (122 mg, 50 %).
NMR spectra should be taken immediately after dissolution in DMSO-d₆ because of exchange of Cl ligands by DMDO-d₆: 1.4 (8 H. m), 1.55 (4 H, m), 1.75 (4 H, m), 2.75 (4 H, t, J = 7 Hz), 3.6 (4 H, t, J = 8 Hz) ppm.

### Example 2

### Preparation of cis-(Bis-(12-amine-1-dodecanol))-dichloroplatinum(II) (1b)

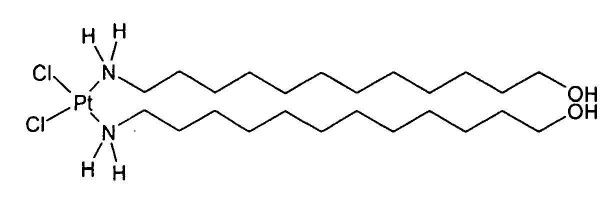

In a 25 ml flask fitted with a magnetic stirrer to 12-aminododecanol (385 mg, 1.91 mmol) 1 N aqueous hydrogen chloride (1.91 ml, 1.91 mmol) was added and the mixture stirred at a bath temperature of 70 °C. Subsequently was added a solution of K₂PtCl₄ (330 mg, 0.79 mmol). Within 2 h, 1 N aqueous NaOH was added dropwise to the beige coloured suspension. In order to avoid the formation of black platinum hydroxide special care was taken not to allow the reaction medium to become alkaline. After addition of 1.58 ml 1N NaOH (1.58 mmol) the suspension had pH 6 and it was allowed to stir for additional 30 min and then to stand at 0 °C for 10 min. The precipitate was collected by centrifugation. It was washed three times with cold 5 % sodium chloride solution (10 ml) and finally with cold water (10 ml). The product was dried in high vacuum affording a beige powder (530 mg, 100 %).
NMR-spectrum in DMF-d₇:1.3 (32 H, broad s), 1.5 (4 H, m), 1.75 (4 H, m), 2.8 (4 H, m) 3.5 (4 H, m), 4.3 (4 H, t, J = 8 Hz), 5.1 (8 H, broad s) ppm.

### Example 3

### Synthesis of cis-(9,10-diamino-1-decanol)-dichloroplatinum(II) (2a)

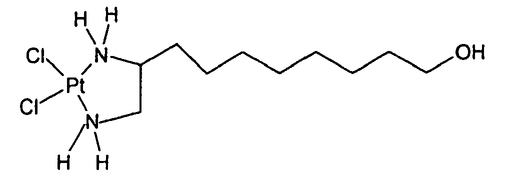

### 1-Benzyloxy-9-decene

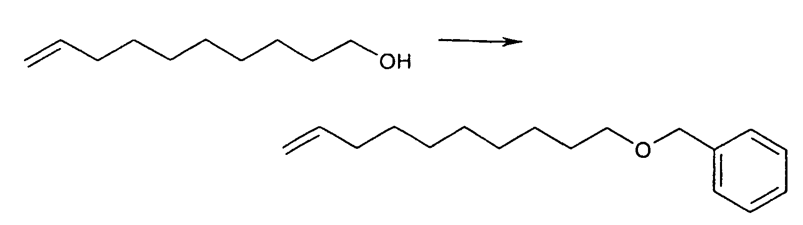

In a three-necked flask fitted with a rubber septum, a magnetic stirrer and a fermentation tube to a suspension of sodium hydride (1.20 g, 5.08 mmol) in dry THF at 0 °C benzyl bromide (7.66 g, 44.8 mmol) was slowly added by a syringe. Subsequently, 9-decene1-ol (7.00 g, 44.8 mmol) was added within 5 min. The ice bath was removed and the mixture was allowed to stir for 14 hr at room temperature, where upon gas evolution came to an end.
The obtained colourless suspension was poured on ice-water (600 ml) and the resulting mixture was extracted twice with portions (400 ml) of ethyl acetate. The organic solutions were washed twice with portions (400 ml) of saturated NaCl solution and then combined and dried over magnesium sulfate. After filtration and evaporation of the solvent in vacuum a yellow oil (12.00 g) resulted. It was purified by column chromatography on silica gel (450 g, 0.063 - 0.200 mm) using toluene (400 ml fractions). From fractions 6 and 7 pure liquid product (10.03 g, 91 %) was obtained. IR spectrum in CH₂Cl₂: 3330, 2930, 2850, 1725, 1635, 1490, 1450, 1360, 1200, 1095, 1025, 995, 910 cm⁻¹.

### 9-Azido-1-benzyloxy-10-iododecane

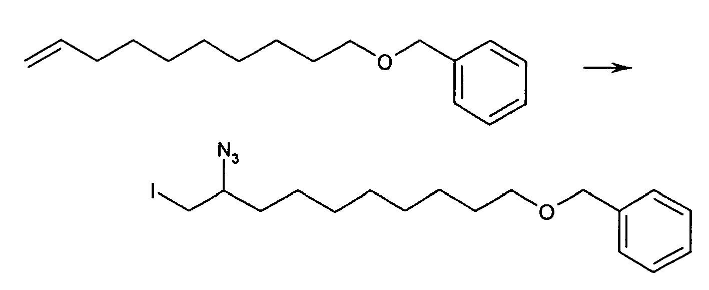

In a dry 25 ml Schlenk flask fitted with a magnetic stirrer and a dropping funnel to a suspension of sodium azide (1.40 g, 21.54 mmol) in dry acetonitrile (3 ml), at 20°C, a solution of iodine chloride (1.54 g, 9.49 mmol) in dry acetonitrile (2 ml) was slowly added within 10 min. The resulting orange-brown solution of iodine azide was allowed to stir for additional 15 min at -15°C. Subsequently, a solution of 1-benzyloxy-9-decene (2.10 g, 8.51 mmol) in dry acetonitrile (2 ml) was added and the dropping funnel rinsed with additional acetonitrile (1 ml). The reaction mixture was ten allowed to stir for three days at room temperature. The dark brown reaction mixture was poured on ice-water (30 ml) and the resulting mixture was extracted three times with portions (10 ml) of ether. The combined organic solutions were washed subsequently with 5 % aqueous sodium thiosulfate solution (20 ml) and three times with portions (10 ml) of water. The organic solution was dried over magnesium sulfate, filtered and the solvent removed in vacuum. The resulting yellow oil was purified by chromatography on silica gel (100 g, 0.063 - 0.200 mm) using cycloxexane/toluene (3 : 1) and 100 ml fractions. From fractions 11 - 22 the product was obtained as colourless oil (2.54 g (72 %). IR spectrum in CH₂Cl₂: 3030, 2940, 2860, 2100, 1455, 1365, 1350, 1260, 1205, 1190, 1105, 1030, 910, 890, 820, 670 cm⁻¹.

### 1-Benzyloxy-9,10-diazidodecane

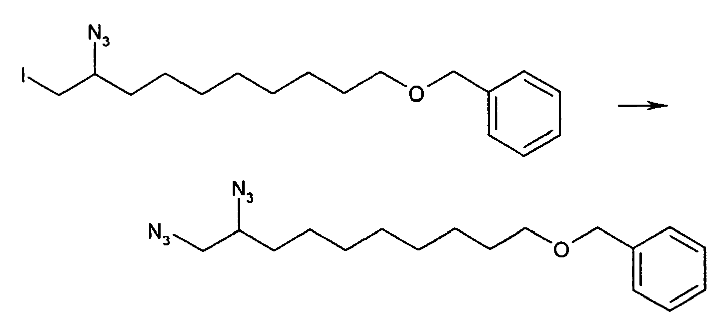

In a dry 10 ml pressure flask to a solution of 9-azido-1-benzyloxy-10-iododecane (1.562 g, 3.76 mmol) in dry DMF (2.5 ml) sodium azide (733 mg, 11.28 mmol) and tetrabutylammonium bromide (73 mg, 0.23 mmol) were added and the flask was sealed and the reaction mixture allowed to stir at a bath temperature of 100°C for 2 days. To the brown suspension water (40 ml) was added and the resulting mixture was extracted three times with portions (40 ml) of ethyl acetate. The organic solutions were combined and washed twice with portions (40 ml) of saturated aqueous sodium chloride solution and then dried over magnesium sulfate. After filtration, the solvent was removed in vacuum affording a brown oil. It was purified by column chromatography on silica gel (40 g, 0.063 - 0.200 mm) using hexane/toluene (2 : 1) and 40 ml fractions. From fractions 7- 22 pure product (958 mg, 77 %) as a colourless oil was obtained. IR spectrum in CH₂Cl₂: 3030, 2930, 2860, 2100, 1715, 1490, 1445, 1350, 1260, 1100, 1030, 915, 660 cm⁻¹.

### 9,10-Diamino-1-decanol dihydrochloride

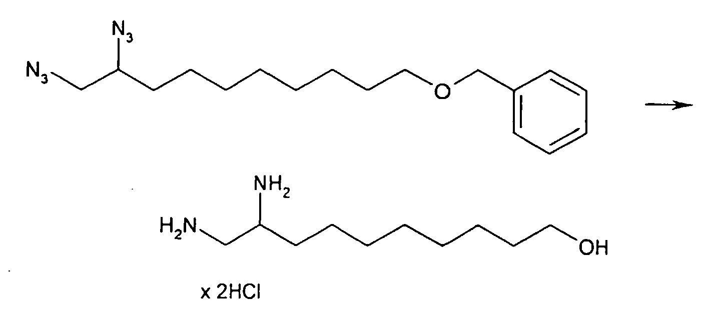

In a hydrogenation apparatus fitted with a magnetic stirrer and a rubber septum 10 % palladium on carbon (520 mg) was prehydrogenated in methanol at room temperature for 10 min. Subsequently, a solution of 1-benzyloxy-9,10-diazidodecane (500 mg, 1.51 mmol) was added by a syringe and the resuting mixture hydrogenated for 1.5 h at room temperature. As nitrogen is formed during this process the uptake of hydrogen could not be observed by volumetry but only by tlc. The apparatus was flushed with nitrogen and to the reaction mixture was given 1 N aqueous HCl (3.02 ml, 3.02 mmol) in order to allow the deprotection of the benzyl ether group by hydrogenolysis. After stirring for 2 min at room temperature hydrogeolysis was continued overnight at room temperature. The uptake of hydrogen was then complete. The catalyst was removed by filtration through a G4 glass filter and washed with methanol (totally 60 ml) and water (totally 60 ml). The solvents were removed in vacuum and the resulting beige residue dried in high vacuum (320 mg, 81 %), mp 125 - 128°C. IR spectrum (KBr): 3340, 3160, 3020, 2930, 2850, 1625,1601, 1569, 1532, 1490, 1465, 1454, 1060, 1040, 1020 cm⁻¹.

### cis-(9,10-Diamino-1-decanol)-dichloroplatinum(II)

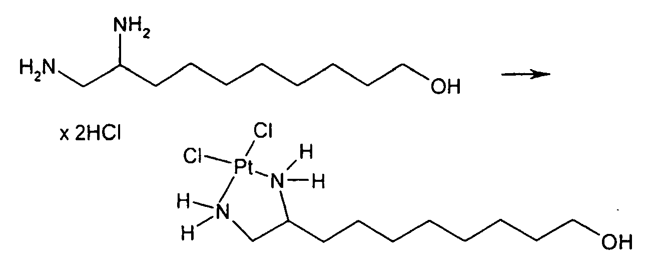

In a 25 ml flask a mixture of 9,10-diamino-1-decanol dihydrochloride (165 mg, 0.63 mmol) in water (5 ml) was heated to 65°C and to the clear beige solution a solution of K₂PtCl₄ (262 mg, 0.63 mmol) in water (2 ml) was added. Slowly, within 2 h, aqueous 1 N NaOH solution was added where upon the red solution was decolourized with the formation of a yellow precipitate. In order to avoid the formation of black platinum hydroxide, special care was taken not to allow the reaction medium to become alkaline. After addition of 1.10 ml 1 N NaOH (1.10 mmol) the suspension had pH 6 and it was allowed to stand at 0 °C for 10 min. The precipitate was collected by centrifugation. It was washed twice with portions (4 ml) of cold water. The product was dried in high vacuum affording a beige powder (228 mg, 79 %), mp >220 °C. NMR spectra should be measured immediately after dissolution in DMSO-d₆ because of exchange of Cl ligands by DMDO-d₆: 1.12 -1.32 (br.s, 10 H), 1.32 - 1.45 (m, 2 H), 1.45 - 1.60 (m 3 H), 2.12 - 2.45 (m, 1 H), 2.52 - 2.72 (m, 1 H), 2.72 - 2.95 (m, 1 H), 3.31 - 3.39 (m, 2 H), 4.41 (br. s, 1 H) 4.98 - 5.60 (m, 4 H).

### Example 4

### 1-((12'-Acetoxydodecyloxy)-methyl)-5-fluorouracil (3a)

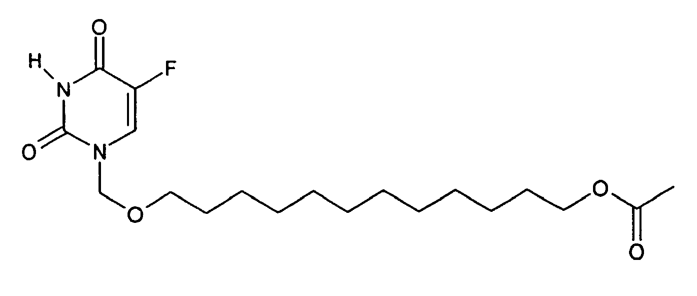

### 12-Acetoxy-1-chloromethoxydodecane

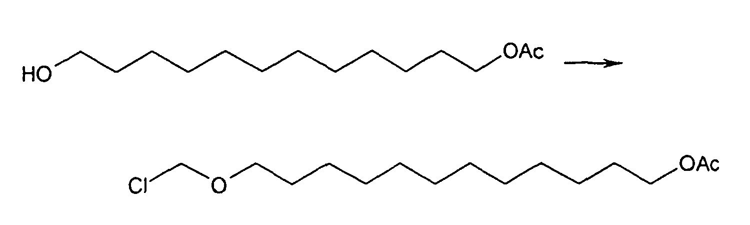

In a dry 10 ml two-necked flask fitted with a gas inlet tube and a magnetic stirrer to a solution of 12-acetoxy-dodecanol (2.61 g, 10.7 mmol) in dry methylene chloride (1.7 ml) methanol free paraformaldehyde (0.32 g, 10.7 mmol) was added and into the obtained suspension at 0 °C dry hydrogen chloride was introduced for 2.5 h. A clear solution resulted. Excess of hydrogen chloride was removed by passing dry nitrogen through the solution for 5 min. Magnesium sulfate was added, filtered and the residue washed with methylene chloride (5 ml). The solvent was removed in vacuum, leaving a yellow oil (2.83 g, 91 %). NMR spectroscopy in CDCl₃ revealed a purity of 66 %. NMR spectrum in CDCl₃: 1.00 -1.48 (m, 16 H), 1.48 - 1.72 (m, 4 H), 2.04 (s, 3H), 3.67 (t, 2 H, J = 6.6 Hz), 4.05 (t, 2 H, J = 6.7 Hz), 5.50 (s, 2 H). The product was used immediately without further purification.

### 1-((12'-Acetoxydodecyloxy)-methyl)-5-fluorouracil

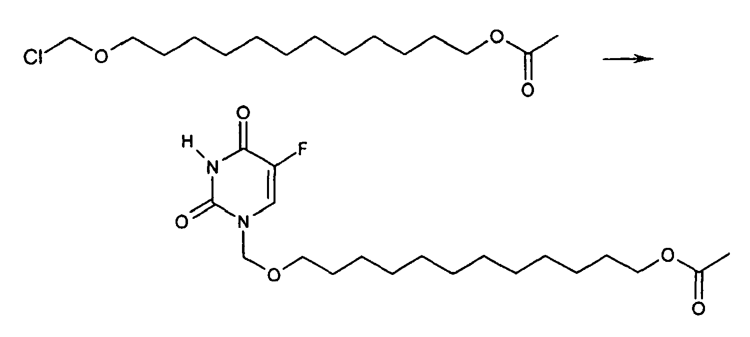

In a dry Schlenk flask fitted with a magnetic stirrer, a rubber septum and a balloon filled with argon to a mixture of the above-mentioned crude 12-acetoxy-1-chloromethoxydodecane (275 mg, 0.62 mmol) and dry toluene (0.5 ml) 2,4-bis-(trimethylsilyloxy)-5-fluoropyrimidine (316 mg, 1.15 mmol) was added by a syringe. After addition the septum was replaced by a dry reflux condenser and the mixture was heated to 100 °C for 2 days. The mixture was diluted with ethyl acetate (10 ml) and then washed twice with portions (10 ml) of aqueous saturated sodium chloride solution. The aqueous layer was back-extracted with ethyl acetate (10 ml). The combined organic solutions were dried with magnesium sulfate, filtered and the solvent removed in vacuum, leaving the crude product as a yellow oil. It was purified by column chromatography over silica gel (12 g, 0.04 - 0.06 mm) using toluene/ethyl acetate (4 : 1) and 10 ml fractions. From fractions 8 - 13 the pure product (118 mg, 50 %) mp 84 - 87°C was obtained. NMR-spectrum in CDCl₃: 0.80 - 1.70 (m, 20 H), 2.05 (s, 3 H), 3.54 (t, 2 H, J = 6.5 Hz), 4.05 (t, 2 H, J = 6.7 Hz), 5.15 (s, 2H), 7.41 (d, 1H, J = 5.2 Hz), 9,79 (br. s, 1 H).

### Example 5

### 5-Fluoro-1-((12'-hydroxydodecyloxy)-methyl)-uracil (3b)

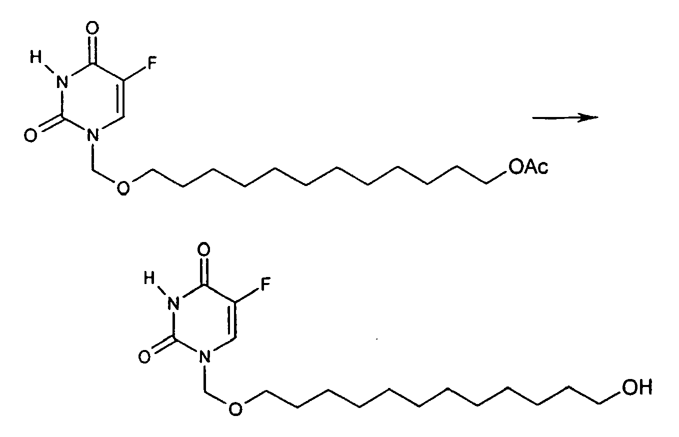

In a 10 ml Schlenk flask, fitted with a rubber septum, a magnetic stirrer and a balloon filled with argon to a slightly turbid solution of 1-((12'-acetoxydodecyloxy)-methyl)-5-fluorouracil (130 mg, 0.34 mmol) in dry methanol (2.2 ml) a 2.2 N solution of sodium methoxide (0.02 ml, 0.44 mmol) in methanol was added by a syringe and the mixture allowed to stir at room temperature for 2 h where upon a clear solution resulted. It was neutralized with 2 N solution of HCl in dry methanol (pH = 4). The solvents were removed in vacuum and the resulting crude product, a colourless solid, containing also sodium chloride, was purified by column chromatography on silica gel (6 g, 0.04 - 0.06 mm) using chloroform/methanol (9 : 1) and 5 ml fractions. From fractions 2 and 3 the pure product (106 mg, 91 %), mp 87 - 89 °C as a colourless powder was obtained. NMR spectrum in CDCl₃: 1.00 - 1.95 (m, 20 H), 2.40 (br. s, 1 H), 3.53 (t, 2 H, J = 6.5 Hz), 3.65 (t, 2 H, 6.6 Hz), 5.13 (s, 2H), 7.39 (d, 1H, J = 5.3 Hz), 9.34 (br. s. 1 H).

### Example 6

### Preparation of [(10-(2,3-Diacetyloxypropyloxy)decyloxy))-methyl]-5-fuorouracil (3c)

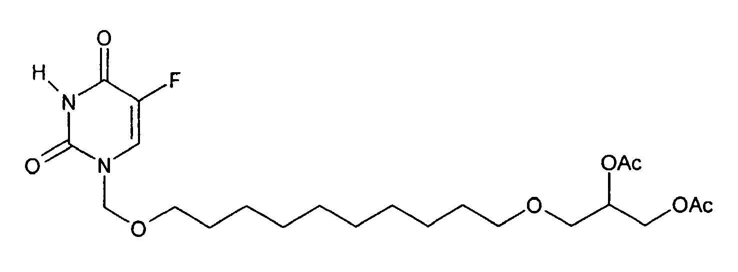

### 10-Undecenyl p-toluenesufonate

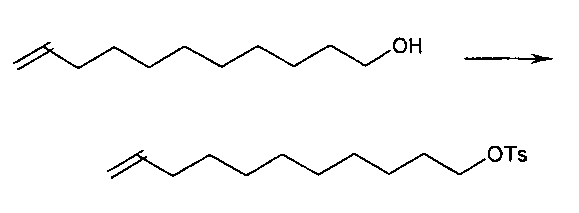

In a dry 25 ml Schlenk flask, fitted with a magnetic stirrer, a rubber septum and a balloon filled with nitrogen to a mixture of 10-undecenol (5.0 ml, 24.1 mmol) and dry pyridine (2.2 ml, 27.3 mmol) tosyl chloride (4.60 g, 24.1 mmol) was added and the suspension stirred at room temperature for 48 h. The viscous reaction mixture was diluted with ether (50 ml) and washed with water (50 ml). The resulting organic layer was subsequently washed with 1 N HCl (20 ml) and twice with portions (50 ml) of saturated NaCl solution. The organic solution was dried over magnesium sulfate, filtered and the solvent removed in vacuum, leaving a yellow oil. It was purified by column chromatography on silica gel (0.063 - 0.200 mm, 210 g) using toluene/ethyl acetate (9 : 1) and 200 ml fractions. From fractions 3 - 10 the pure product was obtained as a pale yellow oil (6.68 g, 85 %). IR spectrum in CH₂Cl₂: 3060, 2930,2860, 1640, 1360, 1195,1180, 1100, 920, 820, 670 cm⁻¹.

### 2,2-Dimethyl-4-(10-undecenyloxymethyl)-1,3-dioxolane

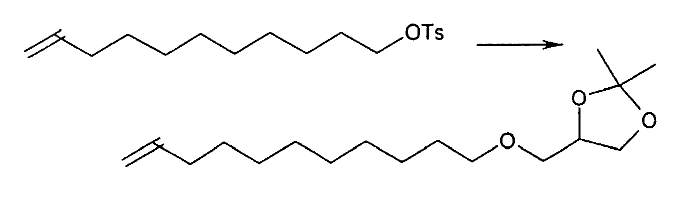

In a 50 ml Schlenk flask, fitted with a magnetic stirrer to a mixture of 10-undecenyl p-toluenesufonate (5.40 g, 16.6 mmol) and DL-isopropylidene glycerol (2.10 ml, 16.9 mmol) in tert-butanol (12.0 ml) potassium tert-butoxide (1.94g, 17.3 mmol) was added in small portions over a 10 min period. The reaction mixture was then allowed to stir at 80 °C for 20 h. The reaction mixture was poured on ice water (100 ml) and was then extracted four times with portions (60 ml) of ether. To obtain phase separation, NaCl was added (approx. 1 g). The combined organic extracts were washed with saturated NaCl solution (100 ml), dried over magnesium sulfate, filtered and the solvent removed in vacuum. The obtained crude product was purified by chromatography on silica gel (0.063 - 0.200 mm, 160g) using 150 ml fractions, first with toluene (fractions 1 - 5) and then with toluene/etthyl acetate (19 : 1, fractions 6 - 20). From fractions 6-10 the product was obtained as a pale yellow oil (3.63 g, 77 %). IR spectrum in CH₂Cl₂: 3690, 3080, 3060, 2990, 2930, 2860, 1640, 1385, 1375, 1245, 1220, 1160, 1120, 1090, 1055 cm⁻¹.

### Racemic 1-O-(10-undecenyl)-glycerol

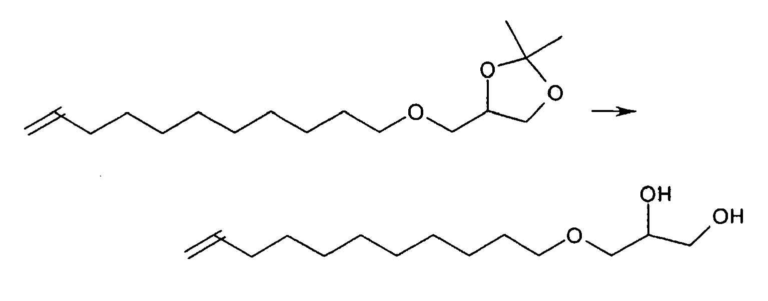

In a 50 ml flask fitted with a magnetic stirrer and a reflux condenser a solution of 2,2-dimethyl-4-(10-undecenyloxymethyl)-1,3-dioxolane (3.39 g, 11.9 mmol) in acetic acid/water (4 : 1, 25 ml) was heated to 65°C for 3 h and then the solvent evaporated in vacuum. The resulting oil was dissolved three times with portions of dry ethanol and evaporated where upon it became partly crystalline. It was then dried in vacuum in a dessicator over silica gel for 24 h. The resulting colourless solid (2.78 g, 96 %), mp. 35 - 37°C, was used without further purification. IR spectrum in CH₂Cl₂: 3690, 3580, 3500, 3070, 3060, 2930, 2860, 1640,1465, 1250, 1120, 1060, 1040, 1000, 920 cm⁻¹.

### Racemic 2,3-Di-O-Acetyl-1-O-(10-undecenyl)-glycerol

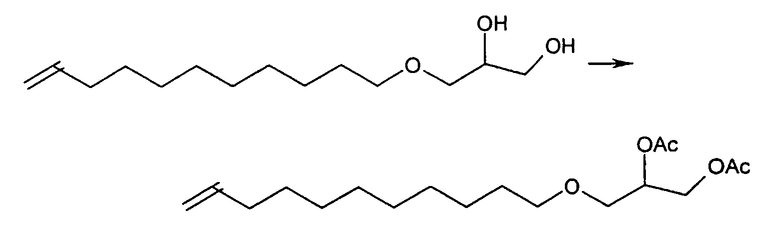

To a solution of *racemic* 1-*O*-(10-undecenyl)-glycerol (1.90 g, 7.77 mmol) in dry pyridine (9.0 ml) acetic anhydride was added and the mixture allowed to stir for 3 h at 70 °C. The reaction mixture was diluted with ethyl acetate and washed with 2 N aqueous HCl (80 ml). The organic phase was then washed with 1 N NaHCO₃ solution and twice with portions (100 ml) of saturated NaCl solution, dried over magnesium sulfate, filtered and the solvent removed in vacuum, leaving a colourless oil (2.49 g, 98 %). IR spectrum in CH₂Cl₂: 3040, 2920, 2850, 1740, 1635, 1455, 1435, 1370, 1225, 1120, 1045, 1015, 960, 910 cm⁻¹.

### Racemic 2,3-Di-O-acetyl-1-O-(10-hydroxydecyl)-glycerol

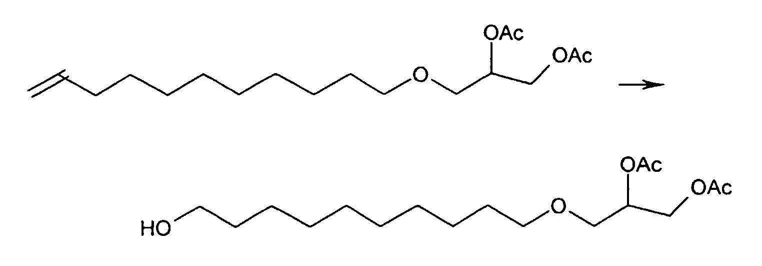

In a two necked 250 ml flask, fitted with a gas inlet tube and a magnetic stirrer into a solution of *racemic* 2,3-Di-*O*-Acetyl-1-*O*-(10-undecenyl)-glycerol (3.14 g, 9.56 mmol) in isopropanol (80 ml) a mixture of ozone and oxygen was introduced at -55 to -50°C. The mixture was then allowed to stand for additional 11 h at 0°C. Tlc indicated a complete conversion. Water (11 ml) was added and then the resulting clear solution was dropwise added to a suspension of sodium borohydride (0.44 g, 11.6 mmol) in isopropanol (30 ml) at 0 °C. The colourless suspension was then allowed to stir overnight at room temperature. After addition of water (100 ml) the reaction mixture was extracted three timrs with portions (150 ml) of methylene chloride. The combined organic solutions were washed with water (100 ml), dried over magnesium sulfate and the solvent removed in vacuum. The obtained crude product was purified over silica gel (0.040 - 0.060 mm, 100 g) using 100 ml fractions and toluene/ethyl acetate (9 : 1, fractions 1 - 7), (4: 1, fractions 8 - 15) and (1 : 1, fractions 16 - 25). From fractions 8 -17 pure product was obtained as a pale yellow oil (2.41 g, 76 %). IR spectrum in CH₂Cl₂: 3620, 3060, 2930, 2860, 1740, 1465, 1375, 1270, 1230, 1125, 1050, 1020 cm⁻¹.

### Racemic 2,3-Di-O-Acetyl-1-O-(10-chloromethoxydecyl)-glycerol

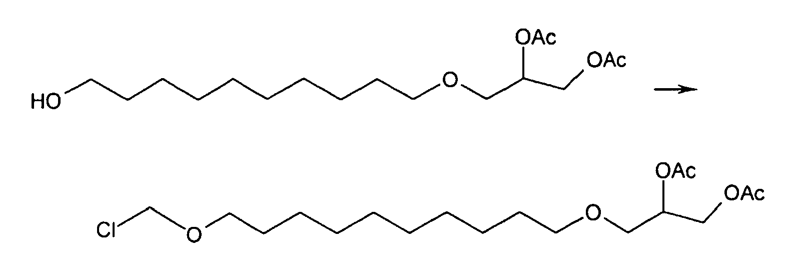

In a dry 10 ml two-necked flask fitted with a gas inlet tube and a magnetic stirrer into a suspension of *racemic* 2,3-Di-*O*-acetyl-1-*O*-(10-hydroxydecyl)-glycerol (1.93 g, 4.16 mmol) and trioxane (0.13 g, 1.44 mmol) at 0 °C dry hydrogen chloride was introduced for 2.5 h. A clear solution resulted. Excess of hydrogen chloride was removed by passing dry nitrogen through the solution for 5 min. Magnesium sulfate and dichloroethane (5 ml) were added, the mixture filtered and the residue washed with dichloroethane (5 ml). The solvent was removed in vacuum, leaving a colourless oil (1.41g, 89 %). NMR spectroscopy in CDCl₃ revealed a purity of 70 %. NMR spectrum in CDCl₃: NMR spectrum in CDCl₃: 1.10 -1.40 (m, 12 H), 1.45 - 1.70 (m, 4 H), 2.06 (s, 3 H), 2.08 (s, 3 H), 3,35 - 3.51 (m, 2 H), 3.54 (d, 2 H, J = 5.3 Hz), 3.67 (t, 2 H, J = 6.5 Hz), 4.16 (dd, 1 H, J = 12.0 Hz, J = 6.4 Hz), 4.33 (dd, 1 H, J = 12.0 Hz, J = 3.8 Hz), 5.18 (dtd, 1 H, J = 6.4 Hz, J = 5.3 Hz, J = 3.8 Hz), 5.50 (s, 2 H). The product was used immediately without further purification.

### [(10-(2,3-Diacetoxypropyloxy)decyloxy))-methyl]-5-fluorouracil

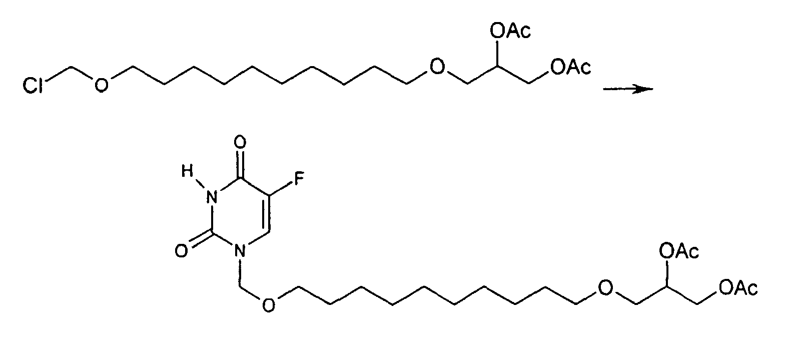

In a dry 10 ml Schlenk flask fitted with a magnetic stirrer, rubber septum and a balloon filled with argon to a solution of 2,4-bis-(trimethylsilyloxy)-5-fluoropyrimidine (1.90 g, 6.92 mmol) in dry toluene (3.0 ml) the above-mentioned crude *racemic* 2,3-Di-*O*-Acetyl-1-*O*-(10-chloromethoxydecyl)-glycerol (1.41 g, 70 % pure, 2.59 mmol) was added by a syringe. The reaction mixture was allowed to stir for 2 h at room temperature. Subsequently, a reflux condenser was attached to the flask and the reaction mixture was allowed to stir for 12 h at 70 °C. Ethanol (0.5 ml) was added, where upon a white precipitate was formed. The solvent was removed in vacuum and the residue purified by chromatography on silica gel (0.063 - 0.200 mm, 100 g) using 100 ml fractions and toluene/ethyl acetate (2 : 1, fractions 1-9) and (1 : 1, fractions 10 - 16). From fractions 6 - 12 the pure product as a pale yellow oil was obtained (928 mg, 76 %). NMR spectrum in CDCl₃: 1.10 - 1.40 (m, 12 H), 1.45 -1.70 (m, 4 H), 2.06 (s, 3H), 2.08 (s, 3H), 3.35 - 3.50 (m, 2 H), 3.53 (t, 2H, J = 6.6 Hz), 3.54 (d, 2H, J = 5.3 Hz), 4.16 (dd, 1 H, J = 11.9 Hz, J = 3.7 Hz), 4.33 (dd, 1 H, J = 11.9 Hz, J = 3.7 Hz), 5.13 (s, 2 H), 5.18 (dtd, 1 H, J = 6.4 Hz, J = 5.3 Hz, J = 3.7 Hz), 7.40 (d, 1 H, J = 5.3 Hz), 9.62 (br. s, 1 H).

### Example 7

### Preparation of [(10-(2,3-Dihydroxypropyloxy)decyloxy))-methyl]-5-fluorouracil (3d)

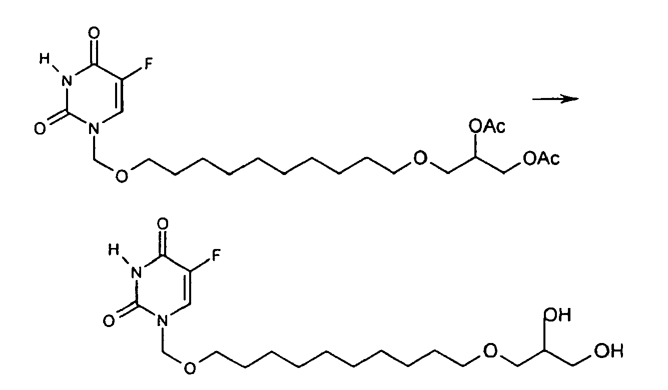

In a 10 ml Schlenk flask, fitted with a rubber septum, a magnetic stirrer and a balloon filled with nitrogen to a solution of [(10-(2,3-diacetoxypropyloxy)decyloxy))-methyl]-5-fluorouracil (385 mg, 0.81 mmol) in dry methanol (1.0 ml) a 1.99 N solution of sodium methoxide (1.05 ml, 2.09 mmol) in methanol was added by a syringe and the mixture allowed to stir at room temperature for 3 h. The solution was neutralized with 2.12 N solution of HCl in dry methanol (pH = 6). The solvents were removed in vacuum and the resulting crude colourless product was suspended in methanol (2 ml) and the solvent removed again in vacuum. The reidue was suspended in ethyl acetate (1 ml) (the insoluble material is sodium chloride) and purified by column chromatography on silica gel (15 g, 0.04 - 0.06 mm) using 12 ml fractions and ethyl acetate (fractions 1 - 6) and ethyl acetate/methanol (fractions 7 - 20). From fractions 5 -10 the pure product (292 mg, 93 %), mp 82 - 84°C as a colourless powder was obtained. NMR spectrum in methanol-d₄: 1.10 - 1.45 (m, 12 H), 1.45 -1.65 (m, 4 H), 3.37 - 3.65 (m, 8 H), 3.68 - 3.80 (m, 1 H), 5.11 (s, 2 H), 7.85 (d, 1 H, J = 6.0 Hz).

### Example 8

### Preparation of cis-(Dichloro-bis-(3-(2,3-dihydroxypropyloxy)propylamine) platinum (II) (3e)

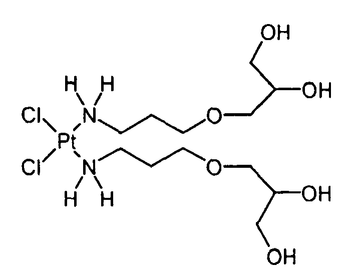

In a 25 ml flask fitted with a magnetic stirrer to 3-*O*-(3-Aminopropyl)-isopropylideneglycerol (189 mg, 1.0 mmol, prepared according to K. Misiura et al. Nucleic Acid Res. 1990, 18, 4351) 2 N aqueous hydrogen chloride (1.5 ml, 3.0 mmol) was added and the mixture allowed to stir at room temperature overnight. Subsequently was added 2 N aqueous KOH (1.5 ml, 3.0 mmol) and a solution of K₂PtCl₄ (200 mg, 0.48 mmol) in water (1 ml). The mixture was allowed to stir at room temperature overnight. The solvent was removed in high vacuum and the resulting yellow waxy solid was extracted with dry methanol in order to remove KCI. After filtration and evaporation of the solvent and drying in high vacuum a yellow solid (190 mg, 85%) was obtained. NMR spectrum in D₃COD: 1.95 (dt, 4H, J = 6.5 Hz), 2.9 (m, 4H, J = 7 Hz), 3.3- 3.9 (m, 14 H), 4.8 (br s, 4H).

### Example 9

### Cytotoxic activity of compounds according to the invention

Experiments were performed in microtiter plates (2 x 10⁴ cells/cm²) at 37°C. After a precultivation period of 24 h the antineoplastic agent was added and cultivation was continued for 72 h. Cell growth was determined by a colorimetric assay based on the conversion of 3-(4,5-dimethyl-2-thiazolyl)-2,5-diphenyltetrazolium (MMT) to a blue formazan product using live mitochondria according to Mosmann, J. Immunolog. Methods 1983, 65, 55.

| Compound | Cell line and cyctotoxic acivity IC₅₀ (µg/ml) | | |
|---|---|---|---|
| | MHEC 5T | LLC | EA.hy 926 |
| 1a | 14 | n.d. | 74 |
| 2a | n.d. | 14 | 40 |
| 3b | 14 | n.d. | 45 |

It is understood that the specification and examples are illustrative but not limitative of the present invention and that other embodiments within the spirit and scope of the invention will suggest themselves to those skilled in the art.

## Claims

1. A tumour selective antineoplastic agent and its pharmaceutically acceptable salts and ester derivatives, **characterized in that** to a customary antineoplastic agent is attached one very specific ω-hydroxyalkyl, (ω-hydroxy)alkenyl, ω-(2,3-dihydroxypropyloxy)alkyl or (ω-(2,3-dihydroxypropyloxy))alkeny group forming a tumour selective conjugate of formula 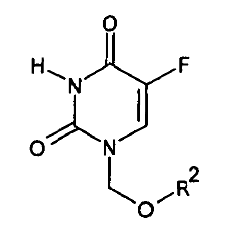 wherein the specific group R² is ω-hydroxyalkyl or (ω-hydroxy)alkenyl both having 5 to 30 carbon atoms, ω-(2,3-dihydroxypropyloxy)alkyl or (ω-(2,3-dihydroxypropyloxy))alkenyl both having 5 to 20 carbon atoms.

2. A tumour selective antineoplastic agent and its pharmaceutically acceptable salts and ester derivatives, **characterized in that** to a customary antineoplastic agent is attached one or two very specific ω-hydroxyalkyl, (ω-hydroxy)alkenyl, ω-(2,3-dihydroxypropyloxy)alkyl or (ω-(2,3-dihydroxypropyloxy))alkenyl groups forming a tumour selective conjugate of formula 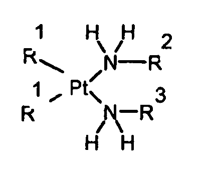 wherein R¹ are either the same or different and are customary pharmaceutically acceptable inorganic or organic leaving groups and R² is unbranched ω-(2,3-dihydroxypropyloxy)alkyl or unbranched (ω-(2,3-dihydroxypropyloxy))alkenyl, both having 5 to 20 carbon atoms and R³ is selected from hydrogen, C₁ - C₆- alkyl, C₃ - C₆- cycloalkyl, unbranched ω-hydroxyalkyl or unbranched (ω-hydroxy)alkenyl both having 5 to 30 carbon atoms, unbranched ω-(2,3-dihydroxypropyloxyalkyl or unbranched (ω-(2,3-dihydroxypropyloxy))alkenyl, both having 5 to 20 carbon atoms.

3. A tumour selective antineoplastic agent and its pharmaceutically acceptable salts and ester derivatives, **characterized in that** to a customary antineoplastic agent is attached one or two very specific ω-hydroxyalkyl, (ω-hydroxy)alkenyl, ω-(2,3-dihydroxypropyloxy)alkyl or (ω-(2,3-dihydroxypropyloxy))alkenyl groups forming a tumour selective conjugate of formula 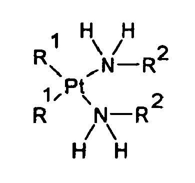 and its pharmaceutically acceptable salts and ester derivatives,
wherein R¹ is a customary pharmaceutically acceptable inorganic or organic leaving group and R² is unbranched ω-hydroxyalkyl or unbranched (ω-hydroxy)alkenyl, both having 5 to 30 carbon atoms, unbranched ω-(2,3-dihydroxypropyloxy)alkyl or unbranched (ω-(2,3-dihydroxypropyloxy))alkenyl, both having 5 to 20 carbon atoms.

4. A tumour selective antineoplastic agent and its pharmaceutically acceptable salts and ester derivatives, **characterized in that** to a customary antineoplastic agent is attached one or two very specific ω-hydroxyalkyl, (ω-hydroxy)alkenyl, ω-(2,3-dihydroxypropyloxy)alkyl or (ω-(2,3-dihydroxypropyloxy))alkenyl groups forming a tumour selective conjugate of formula 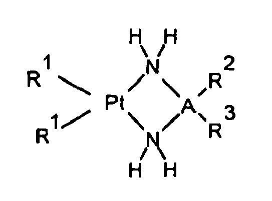 and its pharmaceutical acceptable salts, ester and prodrug derivatives, wherein R¹ are either the same or different and are customary pharmaceutically acceptable inorganic or organic leaving groups and R² is unbranched ω-hydroxyalkyl, unbranched (ω-hydroxy)alkenyl, unbranched ω-(2,3-dihydroxypropyloxy)alkyl or unbranched (ω-(2,3-dihydroxypropyloxy))alkenyl and R³ is selected from hydrogen, alkyl, or C₃ - C₆- cycloalkyl, unbranched ω-hydroxyalkyl, unbranched (ω-hydroxy)alkenyl, unbranched ω-(2,3-dihydroxypropyloxy)alkyl or unbranched (ω-(2,3-dihydroxypropyloxy))alkenyl and where A is 1,2-dimethylene, 1,3-trimethylene, 1,2-cylopentylene or 1,2-cyclohexylene and where the total carbon number of the carbon chain is 5 -30.

5. A compound according to Claim 4 of the formula 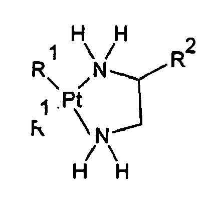 and its pharmaceutically acceptable salts and ester derivatives,
wherein R¹ is a customary pharmaceutically acceptable inorganic or organic leaving group and R² is unbranched ω-hydroxyalkyl, unbranched (ω-hydroxy)alkenyl, unbranched ω-(2,3-dihydroxypropyloxy)alkyl or unbranched (ω-(2,3-dihydroxypropyloxy))alkenyl and where the total carbon number of the carbon chain is 5 - 30.

6. Compounds according to Claims 2 - 5 wherein, independently from each other, R¹ is selected from the group: chloro, bromo, iodo, thiocyanato, nitro, sulfato, nitrato, aquo, hydroxo, malonato, 2-hydoxymalonato, 2-methylmalonato, oxalato, 1,1-cyclopropane-dicarboxylato, 1,1-cyclobutane-dicarboxylato, hydroxyacetato, phenyl-1,2-dicarboxylato, carboxyphenyl-dicarboxylato, sulphophenyl-1,2-dicarboxylato, 2-chloroacetato, 2-bromoacetato and carboxylato with 2 to 20 carbon atoms.

7. A prodrug form of any of the compounds according to Claims 1 - 6, **characterized in that** it is a pharmaceutically acceptable ester derivative or a corresponding Pt (IV) precursor which is metabolized to the active compound in vivo.

8. Use of a compound according to Claims 1 to 7 in the manufacture of a medicament for use in therapy.

9. An antineoplastic composition comprising an antineoplastically effective amount of a compound according to Claims 1 - 7 and a pharmaceutical excipient therefor.

10. An antineoplastic composition according to Claim 9 in unit dose form.

11. A method of using an antineoplastic effective composition according to Claim 9 for the preparation of an anticancer medicament.

## Patentansprüche

1. Ein tumorselektives antineoplastisches Mittel und seine pharmazeutisch annehmbaren Salze und Esterderivate, **dadurch gekennzeichnet, dass** an ein herkömmliches antineoplastisches Mittel eine sehr spezifische ω-Hydroxyalkyl-, (ω-Hydroxy)alkenyl-, w-(2,3-Dihydroxypropyloxy)alkyl- oder w-(2,3-Dihydroxypropyloxy)alkenylgruppe geknüpft wird unter Bildung eines tumorselektiven Konjugates der Formel 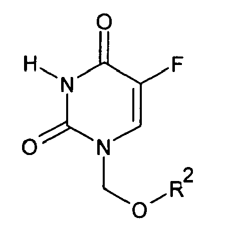 worin die spezifische Gruppe R² ω-Hydroxyalkyl oder (ω-Hydroxy)alkenyl, beide mit 5 bis 30 Kohlenstoffatomen, w-(2,3-Dihydroxypropyloxy)alkyl oder w-(2,3-Dihydroxypropyloxy)alkenyl, beide mit 5 bis 20 Kohlenstoffatomen, bedeuten.

2. Ein tumorselektives antineoplastisches Mittel und seine pharmazeutisch annehmbaren Salze und Esterderivate, **dadurch gekennzeichnet, dass** an ein herkömmliches antineoplastisches Mittel eine oder zwei sehr spezifische w-Hydroxyalkyl-, (w-Hydroxy)alkenyl-, w-(2,3-Dihydroxypropyloxy)alkyl- oder w-(2,3-Dihydroxypropyloxy)alkenylgruppen geknüpft werden unter Bildung eines tumorselektiven Konjugates der Formel 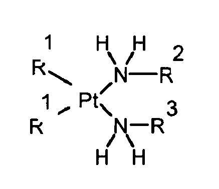 worin R¹ entweder gleich oder verschieden sind und herkömmliche pharmazeutisch annehmbare anorganische oder organische Abgangsgruppen bedeutet und R² unverzweigtes ω-(2,3-Dihydroxypropyloxy)alkyl- oder unverzweigtes (ω-(2,3-Dihydroxypropyloxy))alkenyl, beide mit 5 bis 20 Kohlenstoffatomen, bedeutet und R³ ausgewählt wird aus Wasserstoff, C₁ - C₆-Alkyl, C₃ - C₆-Cycloalkyl, unverzweigtes ω-Hydroxyalkyl oder unverzweigtes (ω-Hydroxy)alkenyl, beide mit 5 bis 30 Kohlenstoffatomen, unverzweigtes w-(2,3-Dihydroxypropyloxy)alkyl oder unverzweigtes (ω-(2,3-Dihydroxypropyloxy))alkenyl, beide mit 5 bis 20 Kohlenstoffatomen.

3. Ein tumorselektives antineoplastisches Mittel und seine pharmazeutisch annehmbaren Salze und Esterderivate, **dadurch gekennzeichnet, dass** an ein herkömmliches antineoplastisches Mittel eine oder zwei sehr spezifische w-Hydroxyalkyl-, (ω-Hydroxy)alkenyl-, ω-(2,3-Dihydroxypropyloxy)alkyl- oder ω-(2,3-Dihydroxypropyloxy)alkenylgruppen geknüpft werden unter Bildung eines tumorselektiven Konjugates der Formel 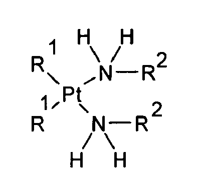 und seine pharmazeutisch annehmbaren Salze und Ester-Derivate, worin R¹ eine herkömmliche pharmazeutisch annehmbare anorganische oder organische Abgangsgruppe und R² unverzweigtes ω-Hydroxyalkyl oder unverzweigtes (ω-Hydroxy)alkenyl, beide mit 5 bis 30 Kohlenstoffatomen, unverzweigtes ω-(2,3-Dihydroxypropyloxy)alkyl oder unverzweigtes (w-(2,3-Dihydroxypropyloxy))alkenyl, beide mit total 5 bis 20 Kohlenstoffatomen bedeuten.

4. Ein tumorselektives antineoplastisches Mittel und seine pharmazeutisch annehmbaren Salze und Esterderivate, **dadurch gekennzeichnet, dass** an ein herkömmliches antineoplastisches Mittel eine oder zwei sehr spezifische ω-Hydroxyalkyl-, (ω-Hydroxy)alkenyl-, w-(2,3-Dihydroxypropyloxy)alkyl- oder ω-(2,3-Dihydroxypropyloxy)alkenylgruppen geknüpft werden unter Bildung eines tumorselektiven Konjugates der Formel 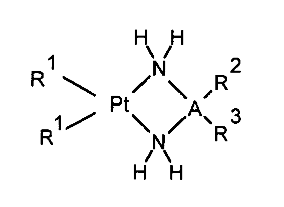 und seine pharmazeutisch annehmbaren Salze, Ester- und Prodrug-Derivate, worin R¹ entweder gleich oder verschieden sind und herkömmliche pharmazeutisch annehmbare anorganische oder organische Abgangsgruppen bedeuten und R² unverzweigtes ω-Hydroxyalkyl, unverzweigtes (ω-Hydroxy)alkenyl, unverzweigtes w-(2,3-Dihydroxypropyloxy)alkyl- oder unverzweigtes (ω-(2,3-Dihydroxypropyloxy))alkenyl bedeutet und R³ ausgewählt wird aus Wasserstoff, Alkyl, C₃ - C₆-Cycloalkyl, unverzweigtes ω-Hydroxyalkyl, unverzweigtes (ω-Hydroxy)alkenyl, unverzweigtes ω-(2,3-Dihydroxypropyloxy)alkyl oder unverzweigtes (ω-(2,3-Dihydroxypropyloxy)alkenyl, und worin A 1,2-Dimethylen, 1,3-Trimethylen, 1,2-Cyclopentylen oder 1,2-Cyclohexylen bedeutet und worin die totale Anzahl Kohlenstoffatome der Kohlenstoffkette 5 - 30 beträgt.

5. Verbindungen gemäß Anspruch 4 der Formel 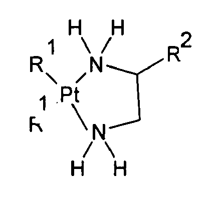 und ihre pharmazeutisch annehmbaren Salze und Ester-Derivate, worin R¹ eine herkömmliche pharmazeutisch annehmbare anorganische oder organische Abgangsgruppe und R² unverzweigtes ω-Hydroxyalkyl-, unverzweigtes (w-Hydroxy)alkenyl, unverzweigtes w-(2,3-Dihydroxypropyloxy)alkyl- oder unverzweigtes w-(2,3-Dihydroxypropyloxy))alkenyl bedeuten und worin die totale Anzahl Kohlenstoffatome der Kohlenstoffkette 5 - 30 beträgt.

6. Verbindungen gemäß Ansprüchen 2 - 5, worin, unabhängig voneinander, R¹ ausgewählt ist aus der Gruppe: Chlor, Brom, Iod, Thiocyanato, Nitro, Sulfato, Nitrato, Aquo, Hydroxo, Malonato, 2-Hydroxymalonato, 2-Methylmalonato, Oxalato, 1,1-Cyclopropan-dicarboxylato, 1,1-Cyclobutan-dicarboxylato, Hydroxyacetato, Phenyl-1,2-dicarboxylato, Carboxyphenyldicarboxylato, Sulphophenyl-1,2-dicarboxylato, 2-Choracetato, 2-Bromacetato und Carboxylato mit 2 bis 20 Kohlenstoffatomen.

7. Eine Prodrugform von irgendeiner Verbindung gemäß der Ansprüche 1 - 6, **dadurch gekennzeichnet, dass** sie ein pharmazeutisch annehmbares Ester-Derivat oder ein entsprechender Pt (IV)-Vorläufer ist, welche in vivo zu der aktiven Verbindung metabolisiert werden.

8. Verwendung einer Verbindung gemäß Ansprüchen 1 bis 7 in der Herstellung eines Medikamentes für den Therapiegebrauch.

9. Eine antineoplastische Zubereitung, die eine antineoplastisch wirksame Menge einer Verbindung gemäß Ansprüchen 1 - 7 und einen pharmazeutischen Träger dafür enthält.

10. Eine antineoplastische Zubereitung gemäß Anspruch 9 in Einheitsdosisform.

11. Eine Methode zur Herstellung eines Anti-Krebs-Medikamentes durch Verwendung einer antineoplastisch wirksamen Zubereitung gemäß Anspruch 9.

## Revendications

1. Un agent antinéoplasique sélectif aux tumeurs, ses sels et dérivés d'ester acceptables d'un point de vue pharmaceutique, **caracterisés en ce qu'**à un agent antinéoplasique et ordinaire, est attaché un groupe très spécifique ω-hydroxyalkyle, (ω-hydroxy)alkényle, ω-(2,3-dihydroxypropyloxy)alkyle ou w-(2,3-dihydroxypropyloxy)alkényle formant un conjugé selectif aux tumeurs de la formule 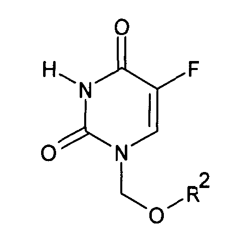 dans laquelle le groupe spécifique R² est ω-hydroxyalkyle ou (w-hydroxy)alkényle, les deux ayant 5 à 30 atomes de carbone, w-(2,3-dihydroxypropyloxy)alkyle ou ω-(2,3-dihydroxypropyloxy)alkényle, les deux ayant 5 à 20 atomes de carbone.

2. Un agent antinéoplasique sélectif aux tumeurs et ses sels et dérivés d'ester acceptables d'un point de vue pharmaceutique, **caracterisés en ce qu'**à un agent antinéoplasique et ordinaire, est attaché un ou deux groupe(s) très spécifique(s) ω-hydroxyalkyle, (ω-hydroxy)alkényle, ω-(2,3-dihydroxypropyloxy)alkyle ou ω-(2,3-dihydroxypropyloxy)alkényle formant un conjugé selectif aux tumeurs de la formule 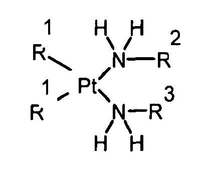 dans laquelle R¹ est identique ou différent et un groupe partant organigue ou inorganique ordinaire, acceptable d'un point de vue pharmaceutique et R² est w-(2,3-dihydroxypropyloxy)alkyle non-ramifié ou w-(2,3-dihydroxypropyloxy)alkényle non-ramifié, les deux ayant 5 à 20 atomes de carbone et R³ est choisi parmi hydrogène, C₁ - C₂-alkyle, C₃ -C₆- cycloalkyle, w-hydroxyalkyle non-ramifié, (w-hydroxy)alkényle non-ramifié, les deux ayant 5 à 30 atomes de carbone, w-(2,3-dihydroxypropyloxy)alkyle non-ramifié ou w-(2,3-dihydroxypropyloxy)alkényle non-ramifié, les deux ayant 5 à 20 atomes de carbone.

3. Un agent antinéoplasique sélectif aux tumeurs et ses sels et dérivés d'ester acceptables d'un point de vue pharmaceutique, **caractérisés en ce qu'**à un agent antinéoplasique et ordinaire est attaché un ou deux groupe(s) très spécifique(s) w-hydroxyalkyle, (w-hydroxy)alkényle, w-(2,3-dihydroxypropyloxy)alkyle ou ω-(2,3-dihydroxypropyloxy)alkényle formant un conjugé selectif aux tumeurs de la formule 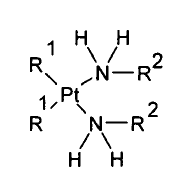 et ses sels et dérivés d'ester; acceptables d'un point de vue pharmaceutique, dans laquelle R¹ est un groupe partant organigue ou inorganique ordinaire, acceptable d'un point de vue pharmaceutique et R² étant ω-hydroxyalkyle non-ramifié, (ω-hydroxy)alkényle non-ramifié, les deux ayant 5 à 30 atomes de carbone, ω-(2,3-dihydroxypropyloxy)alkyle non-ramifié ou w-(2,3-dihydroxypropyloxy)alkényle non-ramifié, les deux ayant 5 à 20 atomes de carbone.

4. Un agent antinéoplasique sélectif aux tumeurs et ses sels et dérivés d'ester, acceptables du point de vue pharmaceutique, **caracterisés en ce qu'**à un agent antinéoplasique et ordinaire, est attaché un ou deux groupe(s) très spécifique(s) ω-hydroxyalkyle, (ω-hydroxy)alkényle, w-(2,3-dihydroxypropyloxy)alkyle ou ω-(2,3-dihydroxypropyloxy)alkényle formant un conjugé selectif aux tumeurs de la formule 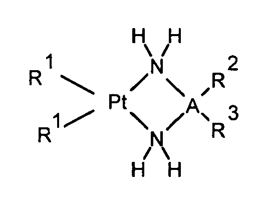 et ses sels, dérivés d'ester et de prodrogue acceptables d'un point de vue pharmaceutique, dans laquelle R¹ est identique ou différent et un groupe partant organigue ou inorganique ordinaire, acceptable d'un point de vue pharmaceutique et R² étant ω-hydroxyalkyle non-ramifié, (ω-hydroxy)alkényle non-ramifié, ω-(2,3-dihydroxypropyloxy)alkyle non-ramifié ou ω-(2,3-dihydroxypropyloxy)alkényle non-ramifié et R³ étant choisi parmi hydrogène, alkyle, C₃ -C₆- cycloalkyle, ω-hydroxyalkyle non-ramifié, (ω-hydroxy)alkényle non-ramifié, ω-(2,3-dihydroxypropyloxy)alkyle non-ramifié ou ω-(2,3-dihydroxypropyloxy)alkényle non-ramifié et dans laquelle A est 1,2-diméthylène, 1,3-triméthylène, 1,2-cyclopentylène ou 1,2-cyclohexylène et dans laquelle le nombre totale d'atomes de carbone de la chaîne varie entre 5 à 30.

5. Composés suivant la revendication 4 de la formule 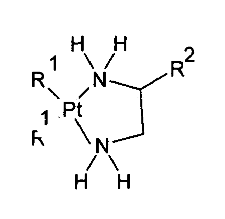 et ses sels et dérivés d'ester, acceptables d'un point de vue pharmaceutique, dans laquelle R¹ est un groupe partant organigue ou inorganique ordinaire, acceptable du point de vue pharmaceutique et R² étant w-hydroxyalkyle non-ramifié, (w-hydroxy)alkényle non-ramifié, ω-(2,3-dihydroxypropyloxy)alkyle non-ramifié ou ω-(2,3-dihydroxypropyloxy)alkényle non-ramifié et dans laquelle le nombre total d'atomes de carbone varie entre 5 à 30.

6. Composés suivant les revendications 2 - 5, où indépendamment l'un de l'autre, R¹ est choisi dans le groupe: chloro, bromo, iodo, thiocyanato, nitro, sulfato, nitrato, aquo, hydroxo, malonato, 2-hydroxymalonato, 2-méthylmalonato, oxalato, 1,1-cyclopropane-dicarboxylato, 1,1-cyclobutane-dicarboxylato, hydroxyacétato, phényl-1,2-dicarboxylato, carboxyphényldicarboxylato, sulphophényl-1,2-dicarboxylato, 2-choroacétato, 2-bromoacétato und carboxylato ayant 2 à 20 atomes de carbone.

7. Une forme prodrogue suivant quelqu'une des revendications 1 - 6, charactérisée en ce que'elle est un dérivé d'ester, acceptable d'un point de vue pharmaceutique ou une précédente correspondant au Pt (IV) qui sont métabolisées formant le composé actif.

8. L'usage d'un composé suivant les revendications 1 à 7 pour la préparation d'un médicament pour l'emploi thérapique.

9. Une composition antinéoplasique comprenant une quantité efficace antinéoplasique d'un composé suivant les revendications 1 - 7 dans un diluant ou un support acceptable du point de vue pharmaceutique.

10. Une composition antinéoplasique de dosage d'unité suivant la revendication 9.

11. Une méthode appliquant une composition efficace antinéoplasique suivant la revendication 9 pour la préparation d'un médicament contre le cancer.
